# EUROPEAN PATENT SPECIFICATION

(11) **EP 1 109 463 B1**
(45) Date of publication and mention of the grant of the patent: **26.11.2003**
(21) Application number: 99945420.0
(22) Date of filing: 01.09.1999
(51) Int. Cl.: A23L 1/36, A23B 4/16, A23B 4/06, A23L 3/015, A23B 4/00, A23B 4/08

(54) **APPARATUS AND METHOD FOR PHYSICALLY MANIPULATING MATERIALS TO REDUCE MICROBE CONTENT**
VORRICHTUNG SOWIE VERFAHREN ZUR PHYSIKALISCHEN BEHANDLUNG VON MATERIALIEN ZUR REDUZIERUNG DES MIKROBENGEHALTES
APPAREIL ET METHODE PERMETTANT DE MANIPULER PHYSIQUEMENT DES MATIERES AFIN D'EN REDUIRE LA TENEUR EN MICROBES

(30) Priority: 01.09.1998 US 144928; 17.12.1998 US 213190; 01.09.1999 US 387979
(43) Date of publication of application: 27.06.2001
(73) Proprietor: Freezing Machines, Inc., Dakota Dunes, South Dakota (US)
(72) Inventor: Roth, Eldon, Dakota Dunes, SD 57049 (US)
(74) Representative: Eder, Thomas, Dr.-Ing.
(86) International application number: US9920112
(87) International publication number: WO00011970

(56) References cited:
- US-A- 3 728 136
- US-A- 4 594 253
- US-A- 4 783 290
- US-A- 5 193 350
- US-A- 5 433 142
- US-A- 5 690 989

## Description

This invention relates to food processing, and more particularly, to reducing microbe content in processed foodstuffs. The invention encompasses both an apparatus and a method for physically manipulating organic materials such as foodstuffs to reduce microbe content.

### BACKGROUND OF THE INVENTION

Foodstuffs are inevitably exposed to microbes in the course of processing or in the course of handling prior to processing. Microbes are part of the natural decay process of organic material and may be deposited on foodstuffs through the air or by contact between the foodstuff and contaminated equipment or other material. Although some microbes may be relatively benign, others contribute to spoilage and some can cause serious illness. Lactic acid producing bacteria are examples of benign microbes, while some strains of E. Coli, Salmonella, Listeria, and Staph bacteria are examples of pathogenic microbes which can cause serious illness when ingested by humans.

Even with careful processing practices, foodstuffs may be exposed to pathogenic microbes during processing or initial handling. Consumers may become ill by ingesting a foodstuff contaminated with pathogenic microbes. However. the risk of illness from dangerous microbes which may be present in foodstuffs is reduced by careful handling and cooking by the consumer. In larger cuts of meat for example, dangerous microbes may only be present on the surface of the meat and are readily killed in the cooking process.

Ground or chopped and mixed foodstuffs, including ground beef, may carry dangerous microbes which are killed only after thoroughly cooking the material. The reason for this is that dangerous microbes residing at the surface of a larger piece of the foodstuff may be distributed throughout the final ground or chopped product as the large piece is ground and mixed together with other pieces. Thorough cooking is required in order to kill microbes residing in the center of a piece of ground and mixed foodstuff.

It is generally desirable to control the growth of microbes and reduce microbe content in foodstuffs. Microbe content and growth in foodstuffs may be reduced by applying chemical additives or preservatives to the foodstuff. These chemical additives or preservatives, however, may not be acceptable to consumers, or may adversely affect the quality of the foodstuffs.

Alternatively to chemical additives or preservatives, heat may be used to kill microbes in foodstuffs. However, heat processing or sterilization often adversely affects the quality or characteristics of the foodstuff and may make the food product undesirable to the consumer.

### SUMMARY OF THE INVENTION

It is an object of the invention to provide a method for reducing microbe content in foodstuffs and to provide an apparatus for performing the method.

The method of the invention comprises physically manipulating or applying stress to the foodstuff while the foodstuff is in a frozen state. While the mechanism by which the process reduces live microbe count is not fully understood, physical manipulation according to the invention has been shown to significantly reduce microbe content in the treated foodstuffs.

The process according to the invention may be performed as a continuous process or as a batch process. In either case, the foodstuff is cooled by a suitable freezing arrangement to a process temperature no greater than the freezing point of the foodstuff to place the foodstuff in a frozen state. As used in this disclosure and the accompanying claims, the "freezing point of the foodstuff" means the temperature at which ice crystals begin to form in the foodstuff at a given pressure. In this "frozen" state according to the invention, liquid material may still be present in the foodstuff along with the ice crystals which have formed. For process temperatures well below the freezing point of the foodstuff, very little or substantially no liquid material may be present in the foodstuff.

Once the foodstuff is frozen, a suitable manipulating arrangement is used to manipulate the frozen material while the material is in a temperature range comprising temperatures not greater than the freezing point of the foodstuff. This physical manipulation according to the invention produces relative movement in the foodstuff. In this sense, "relative movement" means movement between one point in the foodstuff and adjacent points in the foodstuff. It is believed that this relative movement, which preferably occurs throughout the volume of the frozen foodstuff, damages microbes which may be present in the foodstuff and effectively kills much of the live microbes. The invention encompasses manipulating the foodstuff a single time and also manipulating the foodstuff two or more times at substantially the same process temperature or at different temperatures. For example, the foodstuff may be placed at the desired process temperature either from a higher temperature or after being frozen at a lower temperature, and then manipulated once using a suitable manipulating device. After this initial manipulation, the foodstuff may be manipulated again either at the same process temperature or at another process temperature and either with the same type of manipulating device or another type of device. Also, the manipulation steps may be widely spaced apart in time. For example, a comminuted meat product may be manipulated according to the invention one or more times in the production of the product, and may be manipulated again after shipment to another processor or user of the comminuted meat product.

Numerous manipulating arrangements may be employed within the scope of the invention as set out in the following claims. Generally, manipulating arrangements may be classified as (1) compression-type devices, (2) cutting-type devices, or (3) working-type devices. Compression-type manipulating devices include devices which compress the frozen foodstuffs into a block of material, screw-type compressors which compress the frozen foodstuffs as the material is conveyed along a conduit by an auger or screw mechanism, and rolling devices which compress the frozen material as it moves relative to one or more rollers. Cutting-type manipulating devices include grinders. choppers. and slicing devices. Working-type manipulating devices include arrangements which impact the frozen foodstuff and arrangements which bend, stretch, or otherwise work the frozen foodstuff. In each manipulating device, the device may define a working area within which frozen foodstuff is manipulated. As used in the following claims, and unless otherwise specified, a manipulating apparatus or device encompasses any of the types of manipulating devices described in this disclosure. Similarly, a manipulating step may be performed by any of the manipulating devices disclosed herein unless a particular manipulation arrangement is specified.

Regardless of the particular manipulating arrangement employed to manipulate the frozen foodstuffs according to the invention, a manipulator temperature control system associated with the manipulating arrangement preferably controls the temperature of the manipulating arrangement surfaces which come in contact with the frozen foodstuffs. The manipulator temperature control system may cool the surfaces of the manipulating arrangement to ensure that heat from the surfaces of the manipulating arrangement does not raise the temperature of the foodstuffs to a temperature above the desired process temperature. Alternatively, the manipulator temperature control system may heat the manipulating arrangement surfaces and thereby heat the frozen foodstuffs from a lower process temperature to the freezing temperature or even slightly above the freezing temperature. Also, maintaining the surfaces of the manipulating arrangement at a temperature near the process temperature or slightly above the process temperature helps prevent the foodstuffs from sticking to the manipulator surfaces.

One preferred implementation of the invention utilizes a pressure change in a frozen foodstuff to place the foodstuff in a fully or partially unfrozen state. The pressure may be applied as part of a manipulation of the foodstuff or as a separate step. In this aspect of the invention, the foodstuff at a process temperature is compressed by a suitable arrangement to place at least a portion of the foodstuff in a condition in which that portion of the material is not in a frozen state. When the pressure is released, the foodstuff once again goes to a frozen state with ice crystals forming very rapidly.

Physical manipulation according to the invention may be accompanied by manipulating the foodstuff pH before a physical manipulation or both before and after a physical manipulation. The pH of the foodstuff may be modified in any suitable manner. For example, a higher pH foodstuff such as lean finely textured beef may be mixed with a regular ground beef to modify the pH of the resulting mixture. The process of producing lean finely textured beef, which increases pH with respect to the starting material, also represents a suitable pH modifying step. Also, a foodstuff may be placed in contact with NH₃ (Ammonia) in gaseous or aqueous form to increase the pH of the foodstuff. U.S. Patent Application Serial No. 08/803,322, now U.S. Patent No. 5,871,795 discloses a pH modifying apparatus and method which may be employed in this invention. pH modification within the scope of the invention also encompasses decreasing pH. The pH of a foodstuff may be decreased by placing the foodstuff in contact with a pH reducing material such as CO₂, for example.

The present invention is applicable to many types of foodstuffs. For example, comminuted foodstuffs such as ground meats are ideally suited for the present process. The invention may also be applied to larger cuts of meat or cubed or sliced meats. Furthermore, the invention is applicable to vegetable and fruit material including juices. For purposes of this disclosure and the accompanying claims, "foodstuff" may include substantially any food material or mixture of materials which can be held in a workable or manipulatable form at a process temperature. Also, the treatment process is applicable to other materials which contain water and are capable of supporting microbe growth. These other materials are to be considered equivalent to foodstuffs for the purposes of the following claims.

Commonly, the treatment process according to the invention includes forming the material to be treated into a workpiece or a plurality of workpieces either before or after being placed at a process temperature. The workpiece or workpieces may then be physically manipulated by the suitable manipulating device.

These and other objects, advantages, and features of the invention will be apparent from the following description of the preferred embodiments, considered along with the accompanying drawings.

Figure 1 is a diagrammatic representation of a foodstuff pH and physical manipulation system embodying the principles of the invention.

Figure 2 is a partial longitudinal section view of a manipulating arrangement shown diagrammatically in Figure 1.

Figure 3 is a partial transverse section view taken along line 3-3 in Figure 2.

Figure 4 is an enlarged diagrammatic side view of a piece of foodstuff being drawn between the rollers of a preferred manipulating arrangement.

Figure 5 is a view in section taken along line 5-5 in Figure 4.

Figure 6 is a view in section taken along line 6-6 in Figure 4.

Figure 7 is a partial section view showing an alternate manipulating arrangement embodying the principles of the invention.

Figure 8 is a block diagram showing a multiple-cycle treatment process embodying the principles of the invention.

### DESCRIPTION OF THE PREFERRED EMBODIMENTS

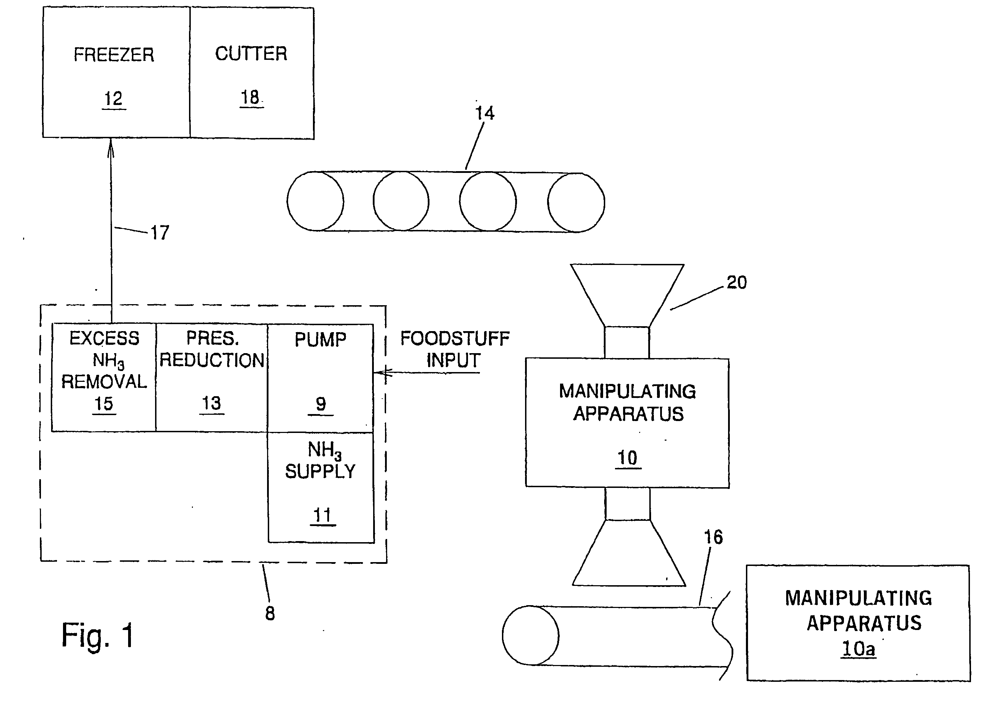

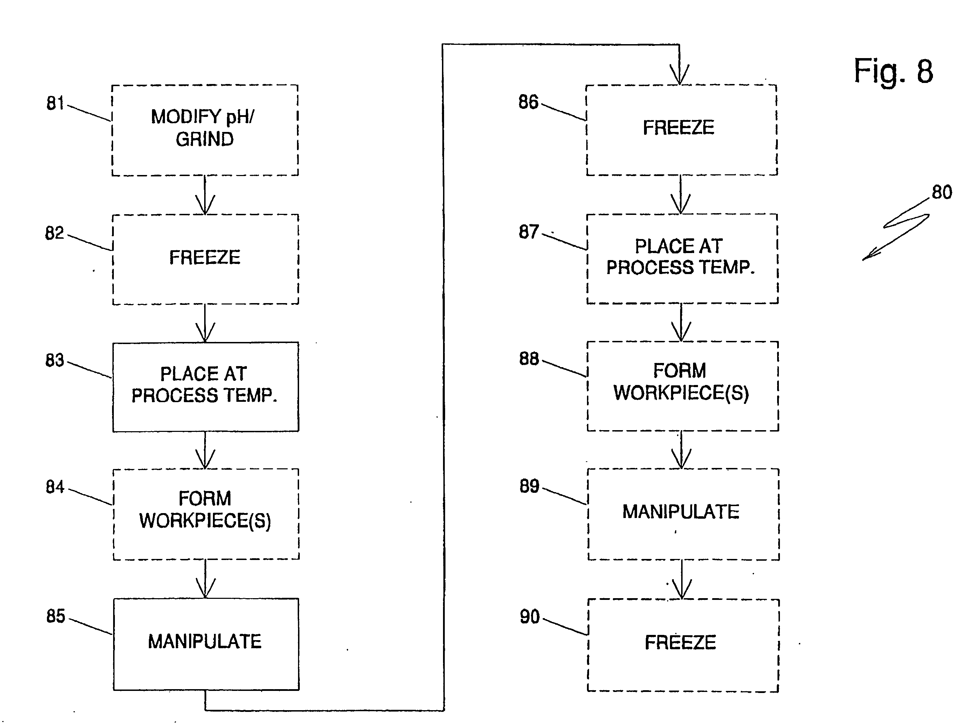

Referring to Figure 1, an apparatus 10 for manipulating foodstuffs is associated with a pH manipulating system 8 and a suitable freezer 12. A transport device 14 is positioned between freezer 12 and manipulating apparatus 10. A second to transport device 16 may be positioned at an outlet from the manipulating apparatus 10 for transporting treated foodstuffs to further processing equipment. An additional manipulating apparatus 10a is shown in Figure 1 for providing an additional manipulation as will be discussed further below with reference to Figure 8.

The illustrated pH manipulating system 8 includes a pump 9, NH₃ supply 11, pressure reduction arrangement 13, and a system 15 for removing excess NH₃. Conduit 17 transfers pH modified foodstuff to freezer 12. Pump 9 preferably comprises a piston pump with an injection arrangement (not shown) for injecting a measured amount of NH₃ from supply 11 into the foodstuff stream either during compression or prior to compression. The pump preferably compresses the NH₃ and foodstuff to a pressure at or above the vapor pressure of the NH₃ at the temperature of the foodstuff. As disclosed in U.S. Patent Application Serial No. 08/803,322, now U.S. Patent No. 5,871,795, this treatmentpressure has been found to rapidly increase the pH of foodstuffs. Pressure reduction arrangement 13 may comprise any suitable device or arrangement for releasing the pressure developed by pump 9. Device 15 may comprise an arrangement for applying a vacuum or any other arrangement for drawing off excess NH₃ after the treatment pressure is released at device 13.

Those skilled in the art will appreciate that many arrangements other than the system 8 shown in Figure 1 may be used to modify the pH of the foodstuff. For example, the foodstuff may be treated in batches in a vessel into which a suitable pH modifying gas is introduced. Also, a liquid material such as aqueous NH₃ may be applied to the foodstuff under pressure or otherwise. Furthermore, an apparatus such as that described in WO 00/059312. entitled "APPARATUS AND METHOD FOR TREATING AMMONIATED MEATS," the disclosure of which is incorporated by this reference, may be used to ensure a consistent pH change throughout the foodstuff to be treated. Any pH modifying material may be used to modify the pH of the foodstuff within the scope of the invention.

Where a pH modification is used along with physical manipulation, the pH change preferably comprises increasing the pH of the foodstuff. However, pH modification within the scope of the invention also encompasses decreasing the pH of the foodstuff. Decreasing the pH may be accomplished by applying a pH decreasing material such as CO₂ gas to the foodstuff preferably under pressure as described above with reference to Figure 1. Furthermore, no pH modifying material may be needed for processing certain foodstuffs. For example, reducing the fat content of a comminuted beef product may change the pH of the material sufficiently within the scope of the invention, as may mixing a reduced fat product with a regular comminuted product.

As will be discussed in further detail below with reference to Figure 8. the pH manipulation step is not mandatory in order to produce the desired microbe kill. Although a pH change prior to physical manipulation may enhance microbe kill in some cases, significant microbe kill is produced by the physical manipulation step (or steps) alone.

In the form of the invention shown in Figure 1, freezer 12 freezes the pH modified foodstuff, and cutter 18 cuts the frozen foodstuff into workpieces which are then placed on transport device 14. Transport device 14 transports the workpieces to manipulating apparatus 10. Manipulating apparatus 10 physically manipulates the frozen foodstuff to produce relative movement between different points in the volume of the foodstuff. This relative movement occurs preferably throughout the entire volume of the foodstuff during the treatment process. It is believed that the relative movement caused by manipulating apparatus 10 damages the cell walls of microbes in the foodstuff, thereby killing the microbes. This damage to the microbes may be accomplished as ice crystals are pressed against the microbe cell walls in the course of the manipulation. The microbes may also become brittle at the processing temperature employed by the invention and the manipulation may serve to damage the cell walls in this relatively brittle state. Although the mechanism by which microbe kill is accomplished is not fully understood, tests of the apparatus and process according to the invention indicate significant microbe kill.

Freezer 12 may be any suitable device capable of cooling the foodstuff to a process temperature no greater than the freezing point of the foodstuff (that is, a temperature less than or equal to the freezing point of the foodstuff). For example, freezer 12 may comprise a roller-type freezer such as the type disclosed in U.S. Patent Nos. 4,138,768 and 4,192,899, which are incorporated herein by this reference. Regardless of the particular type of freezer employed, freezer 12 preferably freezes the foodstuff in less than thirty (30) minutes and optimally in less than about ten (10) minutes. The roller-type freezer disclosed in U.S. Patent Nos. 4,138,768 and 4,192,899 is particularly well-suited for rapidly freezing foodstuffs into thin sheets of material which may then be cut into small sections. Freezing times of approximately 2 minutes may be obtained using these roller-type freezers. Regardless of the freezer type, a rapid first freeze is preferable for purposes of the present invention because rapid freezing produces relatively smaller ice crystals as compared to a slow freezing process. It is believed that the smaller ice crystals improve microbe kill during the manipulation or stressing step according to the invention.

Freezer 12 also preferably has associated with it a forming arrangement for forming the foodstuff into workpieces comprising discrete pieces of foodstuff. The forming arrangement may comprise the cutting system 18 such as the cutting system shown in U.S. Patent No. 4,192,899. This cutting arrangement 18 cuts workpieces from the sheet of frozen foodstuff produced by freezer 12. Alternatively, the foodstuff may be formed into workpieces in an unfrozen state and then frozen to the process temperature. In any event, the workpieces preferably comprise approximately 1.27 x 1.27 cm (half-inch by half-inch) squares having a thickness of approximately 0.635 to 0.317 cm (0.25 to 0.125 inches). Although larger workpieces may be used within the scope of the invention, the thickness of workpieces is preferably less than 0.75 inches. The thinness of the workpiece helps ensure relative movement throughout the volume of the workpiece as it is manipulated according to the invention. Also, thin sheets or workpieces of foodstuff may be cooled more quickly to the process temperature.

Transport device 14 preferably comprises a vibrating conveyor capable of receiving the frozen workpieces from freezer 12 and cutter system 18, and transporting the workpieces to an inlet 20 associated with manipulating apparatus 10. Details of the transport device 14 are not shown in the figures since a number of different types of conveying devices may be employed within the scope of invention, and in any event the details of such conveying devices are well within the knowledge of those skilled in the present field. Transport device 14 preferably moves the frozen workpieces quickly to the manipulating device 10 so that the workpieces are manipulated as quickly as possible after being frozen to the process temperature. It is believed that microbes which have been maintained at the process temperature for a long period of time can better survive the physical manipulation. In the preferred form of the invention, manipulating device 10, freezer 12, and transport device 14 are situated and operated such that the workpieces are manipulated according to the invention as quickly as possible after reaching the process temperature, and preferably no more than about ten (10) minutes, about thirty (30) minutes, about one hour, or as much as 24 hours, after the workpieces reach the process temperature, although longer periods may be used within the scope of the invention.

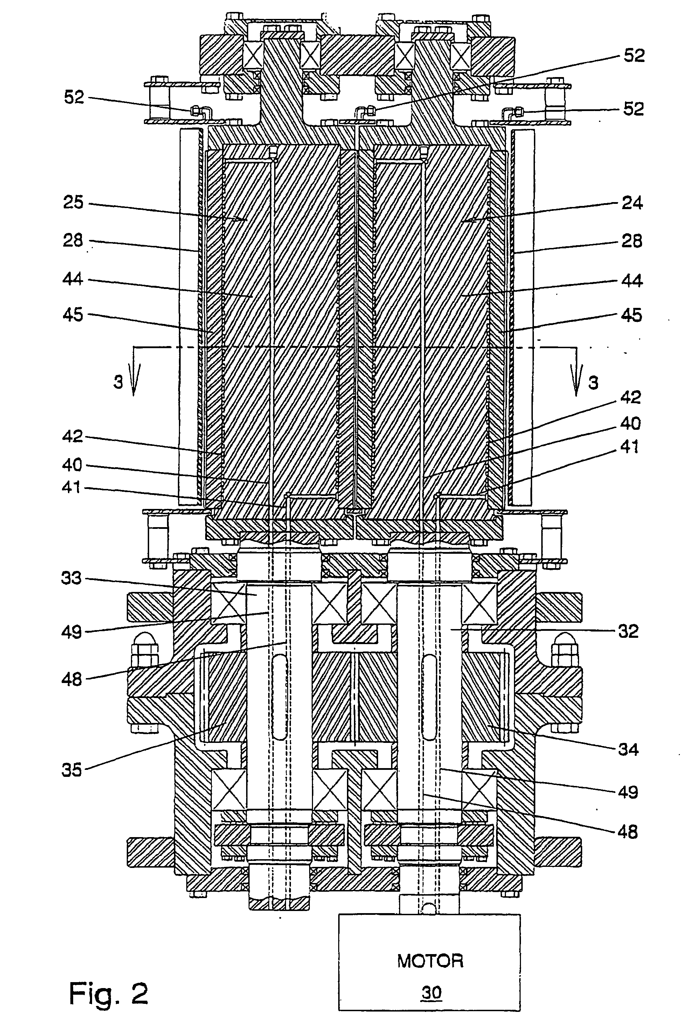

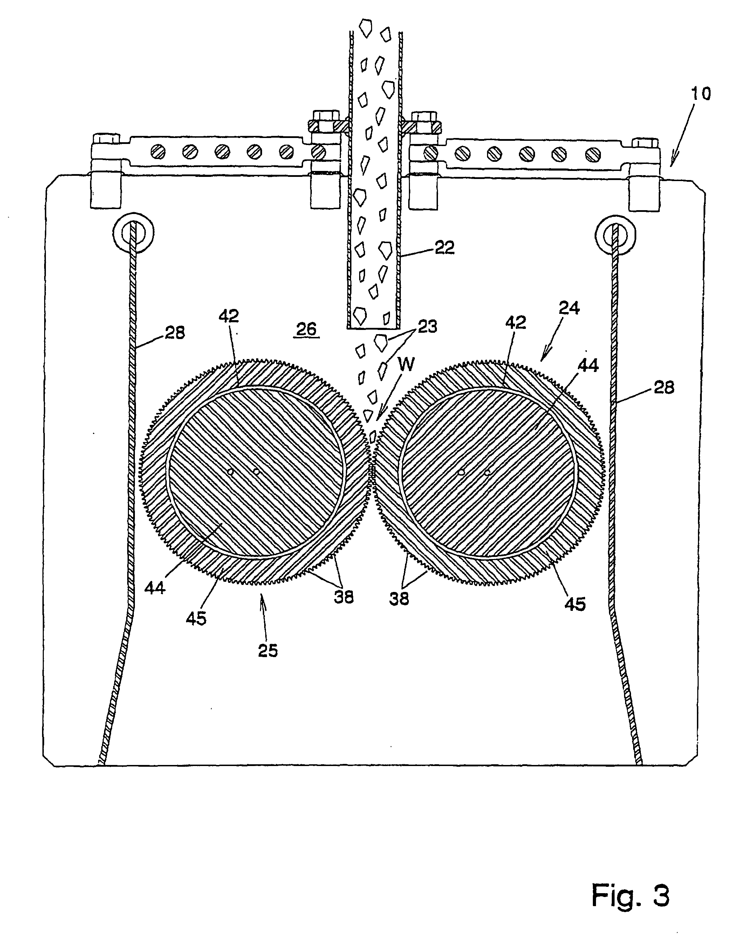

One preferred manipulating apparatus 10 is shown in Figures 2 and 3. Manipulating apparatus 10 includes a chute 22 through which workpieces 23 drop from the transport device 14 shown in Figure 1. The illustrated manipulating apparatus 10 comprises a compression-type device which compresses the foodstuff between two spaced apart rollers 24 and 25 within a chamber 26. Rollers 24 and 25 are positioned within chamber 26 with their longitudinal axes extending substantially parallel to each other. Chamber walls 28 are positioned on either side of the two rollers. Rollers 24 and 25 are spaced apart with a minimum clearance between the roller surfaces which is less than an initial thickness of the workpieces 23. For example, workpieces 23 may be approximately 0.63 to 0.32 cm (0.25 to 0.125 inches) thick and the clearance between the opposing surfaces of rollers 24 and 25 may be approximately 0.25 cm (0.10 inches). The spaced apart rollers 24 and 25 define a working area W extending from the point of minimum clearance between the roller surfaces upwardly to a point at which workpieces 23 first make contact with both rollers.

At least one of the rollers 24 or 25 is driven by a suitable drive motor so as to rotate about its longitudinal axis. The direction of rotation is toward the opposing roller. In the illustrated form of the invention, both rollers 24 and 25 are driven by a single drive motor 30 in a counter rotating fashion toward each other. Drive motor 30 drives first roller 24 directly through shaft 32 and timing gears 34 and 35 cooperate to drive the second roller 25. Timing gear 34 is mounted on drive shaft 32 while timing gear 35 is mounted on drive shaft 33 which is rigidly connected to second roller 25. Although not shown in the drawings, those skilled in the art will appreciate that drive motor 30 includes a suitable transmission arrangement for transmitting power to the first drive shaft 32. The drive motor 30 and associated transmission arrangement may be adapted for driving rollers 24 and 25 at a constant speed, or may be adapted to vary the speed to suit different processing rates.

Although rollers 24 and 25 may have a substantially smooth outer surface, the illustrated preferred rollers include longitudinally extending ridges 38. Rollers 24 and 25 are rotated in synchronization through the timing gears 34 and 35 so that each ridge 38 on one roller registers between adjacent ridges on the opposing roller similarly to the cogs of two intermeshed gears. However, the ridges 38 on the opposing rollers preferably do not touch, but always maintain a minimum clearance between the opposing roller surfaces, for example approximately 0.25 cm (0.10 inches).

The manipulating apparatus 10 shown in Figures 2 and 3 also preferably includes a manipulator temperature control system for cooling or warming the outer surfaces of rollers 24 and 25, that is, the surfaces of the manipulating apparatus which may come in contact with workpieces 23. Maintaining the outer surface of rollers 24 and 25 at a temperature near the process temperature prevents the rollers from heating workpieces 23 above the process temperature as the workpieces come in contact with the roller surfaces. A roller outer surface temperature which is near but just above the particular process temperature being employed also helps prevent workpieces 23 from sticking to the rollers 24 and 25 and may also enhance microbe kill as discussed below. For example, the outer surfaces of the rollers 24 and 25 may be maintained at approximately 0°C (32 degrees Fahrenheit) where the process temperature is approximately -2.2 °C (28 degrees Fahrenheit) or lower.

The illustrated temperature control system includes for each roller 24 and 25 an inlet 40 and an outlet 41. A channel 42 extends near the outer surface of the respective roller and is preferably formed as a continuous spiral groove between an inner roller member 44 and an outer roller member 45. A temperature controlling fluid is circulated through an inlet duct 48 associated with respective roller drive shaft 32 and 33 into the inlet 40, through the continuous spiral channel 42, and out through outlet port 41 and outlet duct 49 formed in the respective drive shaft. Any suitable fluid may be circulated through the temperature control system for cooling or heating the outer surfaces of rollers 24 and 25 preferably to a temperature near the processing temperature, that is, the temperature of the frozen workpieces as they pass between the rollers. The system for producing the temperature control fluid, directing the fluid into inlet duct 48, and receiving the returning fluid from outlet duct 49 is omitted from the drawings so as not to obscure the invention in unnecessary detail. Such system is within the knowledge of those skilled in the art.

The roller-type manipulating apparatus 10 shown in Figures 2 and 3 also preferably includes gas injection ports 52. Any suitable gas such as clean air may be injected through the gas injection ports 52 so as to flow over the outer surfaces of rollers 24 and 25. The injected gas helps clean the roller surfaces and separate foodstuff material which may partially stick to the roller surfaces.

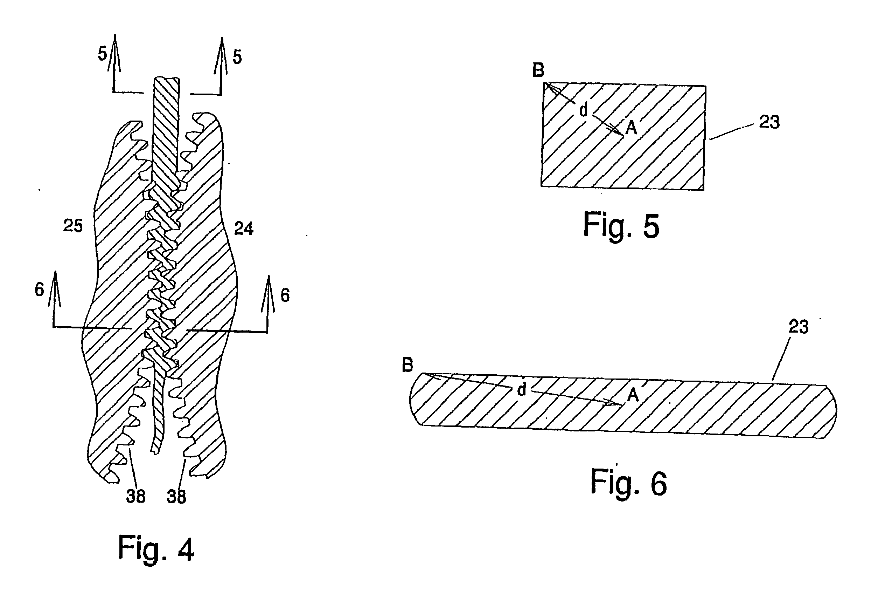

In operation, the roller-type manipulating apparatus 10 shown in Figures 2 and 3 receives a plurality of workpieces 23 into chamber 26 immediately above rollers 24 and 25. As at least one of the rollers is rotated toward the opposite roller or both rollers are rotated toward each other in counter rotating fashion, workpieces 23 are drawn into the working area W and through the area of minimum clearance between the rollers. This action in shown best in Figures 4 through 6. Referring to Figures 4 and 5 each frozen workpiece retains generally its initial shape before passing into the working area W between rollers 24 and 25. However, as shown in Figure 6, the workpiece material is forced to spread out laterally as the workpiece passes through the working area W and ultimately through the area of minimum clearance between rollers 24 and 25. This spreading of the workpiece material causes relative movement between points within the volume of the material. For example, referring to Figure 5, a central point A in workpiece 23 resides a distance d to a point B at one corner of the workpiece. However, as the workpiece 23 spreads out as it passes between rollers 24 and 25 as shown in Figure 6, the distance d between point A and point B changes significantly. This relative movement would occur even if rollers 24 and 25 each had a smooth outer surface. The ridged rollers 24 and 25 have the added advantage of forcing the workpiece material to bend around the opposing ridges 38. This bending in the workpiece material produces additional relative movement between various points within the material.

In the preferred form of the invention, the manipulating arrangement causes relative movement throughout the volume of the foodstuff being processed. Relative movement throughout the foodstuff ensures consistent microbe kill throughout the foodstuff. However, manipulation which produces relative movement in only a portion of the material being processed produces microbe kill in that portion of the material in which the relative movement occurs. Significant microbe kill can be accomplished according to the invention by manipulating the frozen foodstuff so as to produce relative movement in at least approximately twenty percent of the volume of the foodstuff. In the roller-type manipulating arrangement disclosed in Figures 1 through 6, the extent of relative movement in the workpieces is controlled primarily by the clearance between the rollers relative to the initial thickness of the workpieces. A clearance between rollers equal to ninety-five percent (95%) or less of the total initial thickness of the individual workpieces produces the desired compression and relative movement in a significant volume of the foodstuffs being processed.

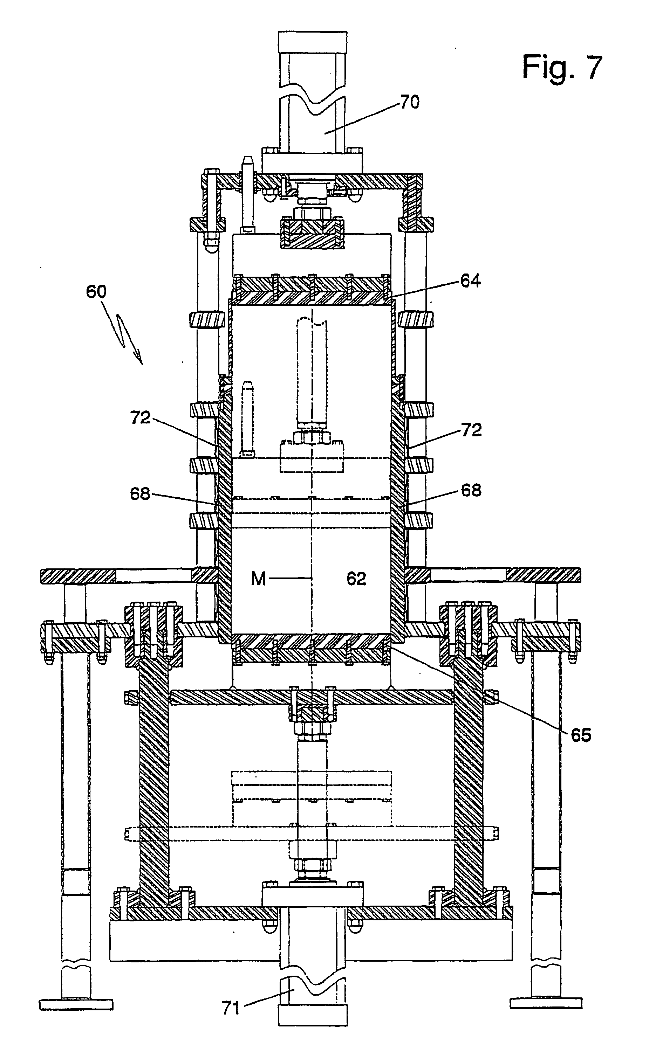

Figure 7 shows a block forming manipulating device 60 according to the invention. In the form of invention shown in Figure 7, workpieces (not shown in Figure 7) are collected in an area 62 bounded by at least one movable plate. The form of invention shown Figure 7 has both a movable top plate or platen 64 and a movable bottom plate or platen 65. Both top plate 64 and bottom plate 65 are movable along axis M relative to chamber walls 68. A top actuator 70 is associated with top plate 64 while a bottom actuator 71 is associated with bottom plate 65. Actuator 70 may comprise a suitable hydraulic or pneumatic piston and cylinder unit for positioning the top plate 64 along the axis M. Actuator 71 may similarly comprise a piston and cylinder arrangement for positioning bottom plate 65 along axis M. Chamber walls 68 may each have a structure 72 which allows a temperature control fluid to be circulated there through for cooling or heating the chamber walls to a temperature near the process temperature similarly to the rollers 24 and 25 discussed above with particular reference to Figures 2 and 3.

In operation, numerous small workpieces (not shown in Figure 7) are randomly arranged in the area 62 defined by bottom plate 65 and chamber walls 68. The random arrangement of rigid small workpieces in the area 62 leaves numerous voids between the individual workpieces. Once the area is filled to a desired point, top actuator 70 is operated to move the top plate downwardly toward bottom plate 65 and into the area 62 defined between the chamber walls 68. A lowered position of top plate 64 is shown in phantom in Figure 7. As top plate 64 advances toward bottom plate 65, the workpieces deform to fill the voids in the volume. This deformation produces relative movement throughout each workpiece.

Top plate 64 may be advanced downwardly until the individual workpieces (not shown in Figure 7) in area 62 deform to produce substantially a solid block of material. At is point, bottom actuator 71 may be operated to move bottom plate 65 downwardly along axis M as top plate 64 continues downwardly. This downward movement of both top plate 64 and bottom plate 65 pushes the block formed from the frozen workpieces out from between chamber walls 68. Once the block clears chamber walls 68, the block of frozen material may be transferred by suitable means to another location for packaging or further processing.

Manipulating apparatus 60 shown in Figure 7 is used in connection with a freezer and a transport device which are not shown in the drawing. The freezer may be the same type of freezer discussed with reference to Figure 1. The transport device may be any suitable conveyor or other device for transporting the frozen, workpieces from the freezer to the chamber area 62. The freezer preferably freezes the workpieces in less than 30 minutes and optimally in less than 10 minutes. Relatively small workpieces are preferable for use in the manipulating arrangement shown in Figure 7. Workpieces having a size on the order of 0.5 inches by 1.27 by 0.63 cm (0.5 inches by 0.25 inches) ensure consistent relative movement within each workpiece. However larger or smaller thin pieces of frozen comminuted or ground foodstuffs may be effectively treated with the apparatus 60 shown in Figure 7.

The block forming manipulating device 60 shown in Figure 7 and the roller manipulating device 10 shown in Figures 2 and 3 are both compression-type manipulating devices. Another compression-type manipulating device may comprise a screw compressor which uses a rotating screw or auger to compress material within a conduit in which the screw or auger is mounted. A screw compressor may force the foodstuff being treated through the openings of a grinder plate or screen and include a cutting arrangement to cut the foodstuff at the grinder plate or screen. Thus, screw compressor manipulating devices may manipulate the foodstuff both by compression and by cutting or grinding. The working area of these screw compressor manipulating devices comprises the area within which the auger rotates.

In addition to screw-type grinders other cutting-type manipulating devices according to the invention include chopping devices such as a bowl chopper which presses a blade repeatedly into a mass of material to chop the material into smaller pieces, and slicing devices which use a blade to slice a relatively thin piece of material from a larger piece. In cutting-type manipulating devices, both a localized pressure and a localized bending action is applied to the frozen foodstuff as the blade or other cutting element passes through the foodstuff. This localized pressure and physical displacement of the foodstuff produces the desired relative movement within the frozen foodstuff and resultant kill of live microbes in the foodstuff. In the bowl cutter device, workpieces of the frozen foodstuff may be fed to the device by any suitable means. In contrast, the slicing device may operate on relatively large pieces of foodstuff at the process temperature, or even a continuous strand of foodstuff fed continuously to the slicing device. These chopping or slicing type manipulating devices must chop or slice the frozen foodstuff into relatively small pieces in order to provide the desired relative movement in a substantial volume of the foodstuff. For example, 1.27 by 1.27 by 0.63 cm (0.5 by 0.5 by 0.25 inch) pieces of frozen foodstuff may be chopped in a bowl chopper in order to produce the desired movement. The material may be chopped in the bowl chopper until the temperature of the material rises to a maximum process temperature no greater than the freezing point of the foodstuff. Slicing-type manipulating devices preferably cut the foodstuff into slices no greater than approximately three-eighths inch thick. The working area comprises the area of the slice adjacent to the blade as it passes through the material.

Working-type manipulating devices according to the invention comprise substantially any arrangement for impacting the frozen foodstuff, or bending, stretching or otherwise working the frozen foodstuff. For example, workpieces of frozen foodstuff may be fed to an impeller arrangement which impacts the workpieces to cause localized pressure increases within the foodstuff and relative movement within the foodstuff as the material deforms in response to the impact. Alternatively, a blending device may produce the desired relative movement as a blending element or paddle passes through a mass of frozen workpieces held in a working area. In this latter case, the workpieces are held at a relatively high process temperature. Such a relatively high process temperature leaves a significant liquid in the foodstuff, causing the material to be relatively malleable.

Bending and stretching a foodstuff at a process temperature may be accomplished by passing the foodstuff between opposing conveyor belts arranged to follow a tortuous path. Alternatively, the foodstuff may be passed through a conduit which follows a tortuous path. In either case, as the foodstuff is bent around a radius comprising the working area, the material at the outside of the radius is placed in tension and stretched somewhat while the material at the inside of the radius is placed in compression. Either condition produces the desired relative movement in the frozen foodstuff, provided the foodstuff is maintained at a relatively high process temperature, in a range from -3.9 °C to 0 °C (25 to 32 degrees Fahrenheit), for example. These relatively high process temperatures leave the foodstuff fairly malleable and capable of being worked.

Figure 8 illustrates the process steps in a multiple-cycle manipulation process 80 according to the invention. The figure may also be used to describe a single manipulation cycle process within the scope of the invention. The process blocks shown in dashed lines are optional and present in the figure only for purposes of fully describing the multiple-cycle aspect of the invention. Also, Figure 8 includes a pH modification step which is optional and not necessary in practicing the physical manipulation process according to the invention.

The first step 81 shown in process 80 is the optional step of modifying the pH of the foodstuff and/or grinding the foodstuff. This first grinding may itself be performed on foodstuff at a process temperature and thus may comprise a first physical manipulation cycle. At optional step 82, the foodstuff is frozen to a relatively low process temperature, preferably quickly using a freezing machine such as a roller-type freezer. It is believed that thoroughly freezing the foodstuff at this point places further strain on live microbes in the foodstuff and ultimately enhances microbe kill.

At step 83 in Figure 8, the foodstuff is placed at the desired process temperature for physical manipulation. The process temperature is preferably from approximately -6.7 °C (20 degrees Fahrenheit) to not greater than -3.9 °C (28 degrees Fahrenheit) for this step. Where step 82 has been performed, step 83 comprises tempering the frozen foodstuff to the desired higher process temperature, either by heating the foodstuff or blending the foodstuff with additional material at a higher temperature. However, where the foodstuff has not been frozen at step 82, step 83 comprises reducing the temperature of the foodstuff by suitable means, preferably using a roller-type freezer described above or a spiral freezer.

Depending upon the type of foodstuff being treated and the type of manipulating device being used, optional step 84 may be included in process 80 to form the foodstuff into suitable workpieces. For example, where the manipulating device comprises the roller-type device 10 shown in Figures 2 and 3 or a block forming device such as device 60 shown in Figure 7, the foodstuff may be cut into pieces on the order of 1.27 by 1.27 by 0.63 cm (0.5 inches by 0.5 inches by 0.25 inches). Where the manipulating device comprises a slicing device or a roller device, the foodstuff may be formed into a substantially continuous strand which is advanced continuously through the respective manipulating device. Such a strand of foodstuff is to be considered equivalent to individual workpieces for purposes of this disclosure and the following claims.

At step 85 the foodstuff at the desired process temperature, formed into appropriate workpieces or otherwise, is manipulated in a suitable manipulating device. The device may comprise a compression-type device, cutting-type device, working-type device, or a device which effectively combines different types of physical manipulation such as a grinder as described above. In each case the manipulating device produces relative movement or displacement within the volume of the foodstuff.

After manipulation at step 85, the foodstuff may be used (cooked, heated for serving, or incorporated into another food product) immediately, or used after storage in a frozen state. Step 86 comprises freezing the manipulated foodstuff, that is, reducing the temperature of the already partially or fully frozen foodstuff. This freezing step after manipulation preferably comprises reducing the temperature of the manipulated foodstuff slowly over the course of one to three days to a temperature of approximately 5 to 10 degrees Fahrenheit. The frozen foodstuff may then be stored or processed through steps 87, 88, 89, and 90 similar to steps 83, 84,85, and 86, respectively. Step 89 represents a second (or third depending on step 81) manipulation step. This multiple-cycle freezing and manipulation process may further reduce the live microbe content of the foodstuff.

The manipulating process according to the invention is particularly applicable to comminuted foodstuffs. As used in this disclosure and in the following claims, a comminuted foodstuff may comprise any ground, chopped, or mixed foodstuff which is made up of relatively small pieces of foodstuffs which have been cut down or otherwise formed from larger pieces. The invention is well suited for treating ground meat such as beef, pork, or poultry. In the following examples, the process was applied to comminuted beef products. However, the invention may be used to treat substantially any foodstuff including comminuted foodstuffs, juices, non-comminuted foodstuffs, and other water bearing materials capable of being manipulated in a frozen or partially frozen state.

The manipulation according to the invention provides an immediate reduction in microbe count. However, microbe count decreases further for a period of time after the manipulation is performed. It is therefore preferable to use the processed foodstuffs no sooner than approximately 24 hours after the manipulation is performed and the material slowly frozen to -15 to -12.2 °C (5 to 10 degrees Fahrenheit). In this sense "use" the foodstuffs means cook or heat the product, or incorporate it into another food product.

Regardless of the particular manipulation arrangement used, it may be desirable to cause at least a portion of each workpiece to go to an unfrozen state during the physical manipulation step. As used in this disclosure and the following claims, an "unfrozen" state means a state in which some, but not necessarily all, ice crystals formed in the foodstuff when the foodstuff was cooled to the process temperature go back to a liquid state. Placing a portion of each workpiece in an unfrozen state may be accomplished by the pressure applied in the manipulation step or may be accomplished by increasing the temperature of a portion of each workpiece during the manipulation step to the freezing point of the foodstuff or slightly above the freezing point. After physically manipulating or stressing the workpieces, each workpiece is re-frozen to a process temperature, that is, a temperature no greater than the freezing point of the foodstuff at the given pressure. The workpieces may be re-frozen by cooling them in a suitable freezing device or by allowing the still frozen portions of the workpieces to re-freeze the adjacent unfrozen portions. Also, re-freezing may be accomplished in some cases simply by removing the pressure applied during the manipulation step. Re-freezing by pressure reduction can be accomplished very quickly and is thus preferred for producing greater stress on live microbes which may be present in the foodstuff.

### EXAMPLE I

A test was performed using a block-type manipulation apparatus similar to that illustrated in Figure 7. Two batches of foodstuffs were processed in the apparatus. A first batch comprised regular ground beef having a pH of approximately 5.25 to 5.5. A second batch was made up of ground beef mixed with 15% (by weight) lean finely textured beef having a pH of approximately 6.25 to 6.5. The ground beef used in the second batch was taken from the same lot as the ground beef used in the first batch. It will be noted that the pH increase in the second batch was accomplished by mixing the higher pH lean finely texture beef with the lower pH ground beef.

The first batch comprising regular ground beef was first processed through a grinder having 0.32 cm (0.125 inch) diameter grinder plate openings. Five samples of the ground beef material were taken at the grinder output, the samples taken approximately 10 seconds apart. The ground beef was then cooled to approximately -2.2 °C (28 degrees Fahrenheit, a temperature below the freezing point of the ground beef material) in about two minutes using a roller-type freezing machine described above. The frozen ground beef was cut into workpieces with a cutting machine associated with the roller-type freezer, the workpieces being about one-eighth inch thick and measuring approximately one-half inch by one-half inch. Five samples were taken at the output of the freezer/cutter arrangement.

The workpieces of ground beef at the process temperature were then placed in a block-type manipulating device similar to that shown in Figure 7. The working area of the device was filled loosely with the workpieces and then the volume of the loose collection of workpieces was reduced by about 50% to form a block of frozen ground beef. The volume reduction was achieved by advancing a top plate. The block of material was then removed from the manipulating device and samples of material were cored at various locations around the block. These cores were mixed together and five samples of frozen material were then collected from the mix of cored material.

Tables 1 through 3 show the results of bacteria tests performed on the various samples described above. Tests were conducted for Total Plate count (TPC), E.Coli count, Coliform count, and Staph count. Tests were also conducted to detect the presence of Salmonella and Listeria. Table 1 shows the results for the five samples taken at the grinder output. Table 2 shows the results for the five samples taken at the freezer/cutter output. Table 3 shows the bacteria test results for the five samples taken from the material removed from the frozen block. Comparing Table 3 to Tables 1 and 2, the freezing and manipulation alone produced a reduction in Coliform and Staph counts in the ground beef.

**TABLE 1**

| Sample | TPC | E. Coli | Coliform | Staph | Sal. | List |
|---|---|---|---|---|---|---|
| 1 | 7,800 | 20 | 150 | 43 | Neg | Neg |
| 2 | 6,000 | 40 | 130 | 23 | Neg | Pos |
| 3 | 13,000 | 10 | 720 | 7 | Neg | Neg |
| 4 | 4,600 | 30 | 490 | 9 | Pos | Neg |
| 5 | 4,700 | 90 | 910 | 43 | Neg | Pos |
| Avg | 7,220 | 38 | 480 | 25 | N/A | N/A |

**TABLE 2**

| Sample | TPC | E. Coli | Coliform | Staph | Sal. | List |
|---|---|---|---|---|---|---|
| 1 | 20,000 | 10 | 250 | 23 | Pos | Neg |
| 2 | 9,200 | 40 | 490 | 4 | Neg | Pos |
| 3 | 16,000 | 10 | 720 | 9 | Neg | Neg |
| 4 | 5,100 | 10 | 130 | 7 | Neg | Neg |
| 5 | 6,900 | 20 | 680 | 23 | Neg | Neg |
| Avg | 11,440 | 18 | 454 | 13.2 | N/A | N/A |

**TABLE 3**

| Sample | TPC | E. Coli | Coliform | Staph | Sal. | List |
|---|---|---|---|---|---|---|
| 1 | 7,600 | 10 | 110 | 9 | Neg | Neg |
| 2 | 5,900 | 20 | 200 | 3 | Neg | Neg |
| 3 | 6,100 | 10 | 270 | 3 | Neg | Pos |
| 4 | 750 | 10 | 210 | 9 | Neg | Neg |
| 5 | 6,700 | 20 | 400 | 3 | Neg | Neg |
| Avg | 5,410 | 14 | 238 | 5.4 | N/A | N/A |

The second batch of beef material, comprising the ground beef mixed with 15 % lean finely textured beef, was processed in the same fashion as the first batch and samples were taken in the same fashion and at the same points. Tables 4 through 6 show the results of bacteria tests performed on the samples from the second batch. Table 4 shows the results for the five samples taken at the grinder output. Table 5 shows the results for the five samples taken at the freezer/cutter output. Table 6 shows the bacteria test results for the five samples taken from the material removed from the frozen block. Comparing the Table 6 test results with those of Tables 4 and 5, there was a marked reduction in Total Plate count, E. Coli count, Coliform count, and Staph count in the samples taken after pH manipulation and manipulation in the block-type manipulation device. The "<" symbol in Table 6 indicates that the observed count was less than the minimum resolution for the test. Also, all tests for Salmonella and Listeria were negative in the material subjected to both pH manipulation and physical manipulation according to the invention.

**TABLE 4**

| Sample | TPC | E. Coli | Coliform | Staph | Sal. | List |
|---|---|---|---|---|---|---|
| 1 | 29,000 | 90 | 720 | 7 | Neg | Neg |
| 2 | 8,600 | 40 | 490 | 4 | Pos | Neg |
| 3 | 13,000 | 180 | 270 | 43 | Pos | Neg |
| 4 | 4,600 | 220 | 1,600 | 23 | Neg | Pos |
| 5 | 6,000 | 110 | 200 | 23 | Neg | Pos |
| Avg | 12,240 | 128 | 656 | 20 | N/A | N/A |

**TABLE 5**

| Sample | TPC | E. Coli | Coliform | Staph | Sal. | List |
|---|---|---|---|---|---|---|
| 1 | 5,800 | 10 | 110 | 9 | Neg | Neg |
| 2 | 3,400 | 40 | 200 | 4 | Neg | Pos |
| 3 | 3,900 | 10 | 270 | 7 | Neg | Neg |
| 4 | 3,100 | 10 | 210 | 4 | Neg | Neg |
| 5 | 5,300 | 20 | 400 | 9.1 | Neg | Neg |
| Avg | 4,300 | 18 | 238 | 6.6 | N/A | N/A |

**TABLE 6**

| Sample | TPC | E. Coli | Coliform | Staph | Sal. | List |
|---|---|---|---|---|---|---|
| 1 | 2,900 | <10 | 10 | <3 | Neg | Neg |
| 2 | 2,700 | <10 | <10 | <3 | Neg | Neg |
| 3 | 2,000 | <10 | 10 | < 3 | Neg | Neg |
| 4 | 300 | <10 | <10 | < 3 | Neg | Neg |
| 5 | 900 | <10 | 10 | <3 | Neg | Neg |
| Avg | 1,760 | <10 | | <3 | N/A | N/A |

### EXAMPLE II

Another test was performed using a block-type manipulation apparatus and a grinder-type manipulation apparatus. The test was performed on a foodstuff comprising lean finely textured beef (LFTB). The LFTB at approximately 10 °C (50 degrees Fahrenheit) was first exposed to ammonia gas to increase the pH of the material. The material was then thoroughly blended with a blending device. After blending, the pH increased LFTB was then frozen on a roller-type freezer to approximately -3.9 °C (25 degrees Fahrenheit) in less than approximately one minute, and cut into pieces measuring approximately 1.27 by 1.27 cm (one-half inch by one-half inch), and approximately 0.63 cm (one-quarter inch) thick. These small workpieces of frozen LFTB were then compressed into a cylinder to form a cylindrically-shaped block of material measuring approximately 5.1 cm (two inches) in diameter by approximately 12.7 cm (five inches) in length. Upon removal of the material from the manipulating device, the temperature of each cylindrically-shaped block of LFTB was reduced slowly over the course of approximately three days to a temperature of approximate -15 to -12.2 °C (five to ten degrees Fahrenheit). The frozen material was then manipulated at approximately -15 to -12.2 °C (five to ten degrees Fahrenheit) with a grinder having a grind size of approximately 0.63 cm (one-quarter inch). The resulting LFTB exiting the grinder was packed into boxes with 28 boxes to a pallet. As each box was being filled, a sample was taken from the box and these samples were mixed with samples from other boxes for the respective pallet to produce a composite mixture. A sample was taken for testing from the composite mixture produced for each pallet. These test samples are numbered 1 through 12 in Tables 7A and 7B. Each sample was tested for total plate count (TPC), and counts for E. Coli, Coliform, Psychrotrophic bacteria, and Staph as shown in Table 7A. Each sample was also tested for Salmonella, Listeria monocytogenes, pH, and ammonia content as shown in Table 7B. The "<" symbol in Table 7A indicates that the observed count was less than the minimum resolution for the test.

**TABLE 7A**

| **SAMPLE** | **TPC** | **E. COLI** | **COLIFORM** | **PSY** | **STAPH** |
|---|---|---|---|---|---|
| 1 | 1110 | <10 | <10 | 2600 | 3.6 |
| 2 | 1400 | <10 | 10 | 1600 | < 3.0 |
| 3 | 1300 | <10 | <10 | 1900 | <3.0 |
| 4 | 5000 | <10 | <10 | 4300 | 39.0 |
| 5 | 3800 | <10 | <10 | 3200 | 9.1 |
| 6 | 3900 | <10 | <10 | 2200 | <3.0 |
| 7 | 2100 | <10 | <10 | 3200 | 9.1 |
| 8 | 3700 | < 10 | <10 | 2300 | 3.0 |
| 9 | 1800 | <10 | <10 | 3400 | 9.1 |
| 10 | 3200 | <10 | 20 | 5300 | 9.1 |
| 11 | 2100 | <10 | 30 | 2700 | <3.0 |
| 12 | 2800 | <10 | <10 | 2900 | 9.1 |

**TABLE 7B**

| **SAMPLE** | **SAL.** | **LIST. MONO**. | **pH** | **NH3%** |
|---|---|---|---|---|
| 1 | NEG | NEG | 6.55 | 0.031 |
| 2 | NEG | NEG | 6.94 | 0.054 |
| 3 | NEG | NEG | 7.02 | 0.077 |
| 4 | NEG | NEG | 7.26 | 0.088 |
| 5 | NEG | NEG | 7.08 | 0.089 |
| 6 | NEG | NEG | 7.07 | 0.076 |
| 7 | NEG | NEG | 7.13 | 0.084 |
| 8 | NEG | NEG | 7.19 | 0.077 |
| 9 | NEG | NEG | 7.62 | 0.100 |
| 10 | NEG | NEG | 7.40 | 0.100 |
| 11 | NEG | NEG | 7.27 | 0.079 |
| 12 | NEG | NEG | 7.20 | 0.068 |

Table 8 shows the results of tests performed on control samples. The control samples were treated as described above, but with no exposure to ammonia. A first control sample was taken from the process stream before the ammonia treatment began and a second control sample was taken after the ammonia treated material was cleared from the system. The results for the tests performed on these control samples are shown in Table 8.

**TABLE 8**

| **CONTROL SAMPLE** | **TPC** | **E. COLI** | **COLIFORM** | **PSY** | **STAPH** | **SAL**. | **LIST. MONO** |
|---|---|---|---|---|---|---|---|
| 1 | 6300 | 50 | 430 | N/S | 23.0 | NEG | POS |
| 2 | 8700 | 210 | 840 | N/S | N/S | N/S | N/S |

## Claims

1. An apparatus for reducing live microbe content in foodstuffs, the apparatus comprising:
(a) a pH modifying arrangement for modifying the pH of an initial foodstuff to produce a pH modified foodstuff;
(b) a freezer for cooling a workpiece to a process temperature no greater than the freezing point of the workpiece, the workpiece comprising a piece of the pH modified foodstuff; and
(c) an initial manipulating arrangement for manipulating the workpiece, the initial manipulation producing relative movement between points within the workpiece while the workpiece remains within a temperature range comprising temperatures no greater than the process temperature.

2. The apparatus of Claim 1 further comprising:
(a) a manipulator temperature system for controlling the temperature of the surfaces of the manipulating arrangement which come in contact with the workpiece.

3. The apparatus of Claim 1 wherein the manipulating arrangement comprises:
(a) a first roller;
(b) a second roller having a rotational axis substantially parallel to a rotational axis of the first roller, the second roller being spaced apart from the first roller such that the clearance between the first roller and second roller is less than an initial thickness of the workpiece so that the workpiece is compressed and allowed to expand laterally as the workpiece is drawn between the first roller and second roller; and
(c) a drive arrangement for driving at least the first roller about its rotational axis toward the second roller.

4. The apparatus of Claim 3 wherein:
(a) the drive arrangement drives both the first roller and the second roller in a counter rotating fashion;
(a) the surface of the first roller and the second roller each include circumferentially spaced apart longitudinal ridges, each ridge on the first roller registering with a space between adjacent ridges on the second roller as the rollers rotate about their respective longitudinal axis.

5. The apparatus of Claim 1 wherein the process temperature is no greater than -2.2 °C (twenty-eight degrees Fahrenheit).

6. The apparatus of Claim 1 wherein the freezer brings the temperature of the pH modified foodstuff from an initial temperature above freezing to the process temperature in no more than thirty minutes.

7. The apparatus of Claim 1 wherein the pH modifying arrangement includes a supply of pH increasing material and an application device within which the initial foodstuff is exposed to the pH increasing material.

8. The apparatus of Claim 7 wherein the pH increasing material comprises NH₃.

9. The apparatus of Claim 1 further comprising:
(a) at least one additional manipulating arrangement, each additional manipulating arrangement for receiving the workpiece after the initial manipulation and manipulating the workpiece, such that each additional manipulation by a respective additional manipulating arrangement produces relative movement between points within the volume of the workpiece while the workpiece remains within a temperature range comprising temperatures no greater than the process temperature.

10. The apparatus of Claim 1 wherein the initial manipulating arrangement comprises:
(a) a first plate spaced apart from a second plate, defining an area between the plates sufficient to receive the workpiece and a plurality of additional workpieces with voids between said workpieces; and
(b) an actuator system for decreasing the distance between the first plate and second plate to manipulate the workpiece and additional workpieces contained there between.

11. The apparatus of Claim 9 wherein the initial manipulating arrangement or at least one additional manipulating arrangement comprises:
(a) a grinder having an auger to compress the workpiece against a grinder screen, and further having a cutting surface driven repeatedly over the grinder screen.

12. A process for decreasing live microbe content in a foodstuff, the process comprising:
(a) modifying the pH of an initial foodstuff to produce a pH modified foodstuff;
(b) cooling a workpiece to a process temperature no greater than the freezing point of the workpiece, the workpiece comprising a piece of the pH modified foodstuff; and
(c) manipulating the workpiece while the workpiece is maintained within a temperature range comprising temperatures no greater than the freezing point of the workpiece, the manipulation producing relative movement between points within the workpiece;

13. The method of Claim 12 further comprising the step of :
(a) controlling the temperature of surfaces which come in contact with the workpiece when the workpiece is manipulated.

14. The method of Claim 12 wherein the step of modifying the pH of the initial foodstuff to be processed to produce the pH modified foodstuff comprises:
(a) placing the initial foodstuff in contact with NH₃.

15. The method of Claim 12 further comprising the steps of:
(a) increasing the pressure on the workpiece as the workpiece is manipulated to cause at least a portion of the workpiece to go to an un-frozen state as the workpiece is manipulated; and
(b) placing back in a frozen state that portion of the workpiece which went to an un-frozen state as the workpiece was manipulated.

16. The method of Claim 12 wherein the step of manipulating the workpiece comprises:
(a) passing the workpiece between a first roller and a second roller, the second roller being spaced apart from the first roller such that the clearance between the first roller and second roller is less than an initial thickness of the workpiece so that the workpiece is compressed and allowed to expand laterally as it advances between the first roller and second roller.

17. The method of Claim 12 wherein the step of manipulating the workpieces comprises:
(a) placing the workpiece and a plurality of additional workpieces between a first plate and a second plate; and
(b) decreasing the distance between the first plate and second plate to compress the workpiece and additional workpieces.

18. The process of Claim 12 further comprising the steps of:
(a) modifying the temperature of the workpiece to a different temperature no greater than the freezing point of the workpiece; and
(b) manipulating the workpiece in at least one additional manipulation while the workpiece is maintained within a temperature range comprising temperatures no greater than the freezing point of the workpiece, the manipulation producing relative movement between points within the workpiece.

## Patentansprüche

1. Vorrichtung zur Verringerung des Anteils lebender Mikroben in Lebensmitteln, wobei die Vorrichtung Folgendes aufweist:
a) eine pH-Modifikationsanordnung, um den pH-Wert eines Ausgangslebensmittels so zu modifizieren, dass ein pH-modifiziertes Lebensmittel entsteht;
b) eine Gefriereinrichtung, um ein Bearbeitungsobjekt auf eine Prozesstemperatur abzukühlen, die nicht höher ist als der Gefrierpunkt des Bearbeitungsobjekts, wobei das Bearbeitungsobjekt aus einem Stück des pH-modifizierten Lebensmittels besteht; und
c) eine Anfangsbehandlungsanordnung zur Behandlung des Bearbeitungsobjekts, wobei bei der anfänglichen Behandlung eine relative Bewegung von Punkten innerhalb des Bearbeitungsobjekts zueinander erfolgt, während das Bearbeitungsobjekt in einem Temperaturbereich bleibt, der Temperaturen umfasst, die nicht höher sind als die Prozesstemperatur.

2. Vorrichtung nach Anspruch 1, die außerdem aufweist:
a) ein Behandlungstemperatursystem zur Steuerung der Temperatur der Oberflächen der Behandlungsanordnung, die mit dem Bearbeitungsobjekt in Kontakt kommen.

3. Vorrichtung nach Anspruch 1, wobei die Behandlungsanordnung Folgendes aufweist:
a) eine erste Rolle;
b) eine zweite Rolle, deren Rotationsachse im Wesentlichen parallel zu einer Rotationsachse der ersten Rolle ist, wobei die zweite Rolle von der ersten Rolle so beabstandet ist, dass die Lücke zwischen der ersten Rolle und der zweiten Rolle kleiner ist als eine anfängliche Dicke des Bearbeitungsobjekts, so dass das Bearbeitungsobjekt zusammengedrückt wird und sich seitlich ausdehnen kann, wenn das Bearbeitungsobjekt zwischen der ersten Rolle und der zweiten Rolle durchgezogen wird; und
c) eine Antriebsanordnung, um zumindest die erste Rolle um ihre Rotationsachse zur zweiten Rolle hin anzutreiben.

4. Vorrichtung nach Anspruch 3, bei der:
a) die Antriebsanordnung sowohl die erste Rolle als auch die zweite Rolle im Gegenlauf antreibt;
b) die Oberfläche der ersten Rolle und der zweiten Rolle jeweils entlang dem Umfang voneinander beabstandete longitudinale Rippen aufweist, wobei jede Rippe auf der ersten Rolle passgenau in einen Leerraum zwischen benachbarten Rippen auf der zweiten Rolle greift, wenn die Rollen sich um ihre jeweilige Längsachse drehen.

5. Vorrichtung nach Anspruch 1, bei der die Prozesstemperatur nicht höher ist als -2,2°C (28 Grad Fahrenheit).

6. Vorrichtung nach Anspruch 1, bei der die Gefrieranlage die Temperatur des pH-modifizierten Lebensmittels in nicht mehr als dreißig Minuten von einer Anfangstemperatur über dem Gefrierpunkt bis zur Prozesstemperatur bringt.

7. Vorrichtung nach Anspruch 1, bei der die pH-Modifikationsanordnung eine Zufuhr von den pH-Wert erhöhendem Material und eine Anwendungsvorrichtung aufweist, in der das Ausgangslebensmittel dem den pH-Wert erhöhenden Material ausgesetzt wird.

8. Vorrichtung nach Anspruch 7, bei der das den pH-Wert erhöhende Material NH₃ aufweist.

9. Vorrichtung nach Anspruch 1, die außerdem aufweist:
a) mindestens eine zusätzliche Behandlungsanordnung, wobei jede zusätzliche Behandlungsanordnung das Bearbeitungsobjekt nach der anfänglichen Behandlung zugeführt erhält und das Bearbeitungsobjekt so behandelt, dass bei jeder zusätzlichen Behandlung durch eine jeweilige zusätzliche Behandlungsanordnung eine relative Bewegung zwischen Punkten innerhalb der Masse des Bearbeitungsobjekts erzeugt wird, während das Bearbeitungsobjekt in einem Temperaturbereich bleibt, der Temperaturen umfasst, die nicht höher sind als die Prozesstemperatur.

10. Vorrichtung nach Anspruch 1, bei der die Anfangsbehandlungsanordnung Folgendes aufweist:
a) eine erste Platte, die von einer zweiten Platte beabstandet ist, wobei ein Bereich zwischen den Platten definiert wird, der ausreicht, um das Bearbeitungsobjekt sowie eine Mehrzahl zusätzlicher Bearbeitungsobjekte mit Lücken zwischen den Bearbeitungsobjekten aufzunehmen; und
b) ein Betätigungssystem, mit dem der Abstand zwischen der ersten Platte und der zweiten Platte verringert wird, um das Bearbeitungsobjekt und zusätzliche Bearbeitungsobjekte, die dazwischen angeordnet sind, zu behandeln.

11. Vorrichtung nach Anspruch 9, bei der die Anfangsbehandlungsanordnung oder mindestens eine zusätzliche Behandlungsanordnung Folgendes aufweist:
a) einen Zerkleinerer mit einer Förderschnecke, um das Bearbeitungsobjekt gegen einen Zerkleinerungsschirm zu drücken, und mit einer Schneidefläche, die wiederholt über den Zerkleinerungsschirm bewegt wird.

12. Verfahren zur Verringerung des Anteils lebender Mikroben in einem Lebensmittel, wobei das Verfahren Folgendes aufweist:
a) Modifikation des pH-Werts eines Ausgangslebensmittels, um ein pH-modifiziertes Lebensmittel zu erzeugen;
b) Abkühlen eines Bearbeitungsobjekts auf eine Prozesstemperatur, die nicht höher ist als der Gefrierpunkt des Bearbeitungsobjekts, wobei das Bearbeitungsobjekt aus einem Stück des pH-modifizierten Lebensmittels besteht; und
c) Behandeln des Bearbeitungsobjekts, während das Bearbeitungsobjekt in einem Temperaturbereich gehalten wird, der Temperaturen umfasst, die nicht höher sind als der Gefrierpunkt des Bearbeitungsobjekts, wobei bei der Behandlung eine relative Bewegung von Punkten innerhalb des Bearbeitungsobjekts zueinander erzeugt wird.

13. Verfahren nach Anspruch 12, das außerdem folgenden Schritt aufweist:
a) Steuern der Temperatur von Oberflächen, die mit dem Bearbeitungsobjekt in Kontakt kommen, wenn das Bearbeitungsobjekt behandelt wird.

14. Verfahren nach Anspruch 12, bei dem der Schritt der Modifikation des pH-Werts des zu verarbeitenden Ausgangslebensmittels zur Erzeugung des pH-modifizierten Lebensmittels Folgendes aufweist:
a) In-Kontakt-Bringen des Ausgangslebensmittels mit NH₃.

15. Verfahren nach Anspruch 12, das außerdem folgende Schritte aufweist:
a) Erhöhen des Drucks auf das Bearbeitungsobjekt, wenn das Bearbeitungsobjekt behandelt wird, um zu bewirken, dass zumindest ein Teil des Bearbeitungsobjekts in einen nicht gefrorenen Zustand übergeht, wenn das Bearbeitungsobjekt behandelt wird; und
b) Zurückversetzen des Teils des Bearbeitungsobjekts, der bei der Behandlung in einen nicht gefrorenen Zustand versetzt wurde, in einen gefrorenen Zustand.

16. Verfahren nach Anspruch 12, bei dem der Schritt der Behandlung des Bearbeitungsobjekts Folgendes umfasst:
a) Transportieren des Bearbeitungsobjekts zwischen einer ersten Rolle und einer zweiten Rolle, wobei die zweite Rolle so von der ersten Rolle beabstandet ist, dass die Lücke zwischen der ersten Rolle und der zweiten Rolle kleiner ist als die Ausgangsdicke des Bearbeitungsobjekts, so dass das Bearbeitungsobjekt zusammengedrückt wird und sich seitlich ausbreiten kann, wenn es zwischen der ersten Rolle und der zweiten Rolle vorwärts bewegt wird.

17. Verfahren nach Anspruch 12, bei dem der Schritt der Behandlung der Bearbeitungsobjekte Folgendes aufweist:
a) Anordnen des Bearbeitungsobjekts und einer Mehrzahl von zusätzlichen Bearbeitungsobjekten zwischen einer ersten Platte und einer zweiten Platte; und
b) Verringern des Abstands zwischen der ersten Platte und der zweiten Platte, um das Bearbeitungsobjekt und zusätzliche Bearbeitungsobjekte zusammenzudrücken.

18. Verfahren nach Anspruch 12, das außerdem folgende Schritte aufweist:
a) Modifizieren der Temperatur des Bearbeitungsobjekts auf eine andere Temperatur, die nicht höher ist als der Gefrierpunkt des Bearbeitungsobjekts; und
b) Behandeln des Bearbeitungsobjekts durch mindestens eine zusätzliche Behandlung, während das Bearbeitungsobjekt in einem Temperaturbereich gehalten wird, der Temperaturen umfasst, die nicht höher sind als der Gefrierpunkt des Bearbeitungsobjekts, wobei die Behandlung eine relative Bewegung von Punkten im Inneren des Bearbeitungsobjekts zueinander bewirkt.

## Revendications

1. Appareil visant à réduire la teneur en microbes vivants dans des denrées alimentaires, l'appareil comprenant :
(a) un dispositif de modification du pH pour la modification du pH d'une denrée alimentaire initiale pour produire une denrée alimentaire à pH modifié ;
(b) un réfrigérateur pour le refroidissement d'une pièce à une température de traitement inférieure au point de congélation de la pièce, celle-ci comprenant un morceau de la denrée alimentaire à pH modifié ; et
(c) un dispositif de manipulation initial pour la manipulation de la pièce, la manipulation initiale produisant le mouvement relatif entre les points au sein de la pièce, tandis que la pièce demeure dans une gamme de températures comprenant des températures inférieures à la température de traitement.

2. Appareil de la revendication 1 comprenant en outre :
(a) un système de contrôle de température de manipulation pour la commande de la température des surfaces du dispositif de manipulation qui entrent en contact avec la pièce.

3. Appareil de la revendication 1 dans lequel le dispositif de manipulation comprend :
(a) un premier rouleau ;
(b) un second rouleau ayant un axe de rotation sensiblement parallèle à un axe de rotation du premier rouleau, le second rouleau étant espacé du premier rouleau de telle sorte que le dégagement entre le premier et le second rouleau est inférieur à une épaisseur initiale de la pièce, si bien que la pièce est comprimée et peut s'étendre latéralement lorsque la pièce est tirée entre le premier et le second rouleaux, et
(c) un dispositif d'entraînement pour entraîner au moins le premier rouleau sur son axe de rotation vers le second rouleau.

4. Appareil de la revendication 3, dans lequel :
(a) le dispositif d'entraînement entraîne à la fois le premier et le second rouleaux selon une rotation antagoniste ;
(b) les surfaces du premier rouleau et du second rouleau comprennent respectivement des nervures longitudinales espacées les unes des autres de façon circonférentielle, chaque nervure située sur le premier rouleau correspondant à un espace entre les nervures adjacentes situées sur le second rouleau lorsque les rouleaux tournent autour de leur axe longitudinal respectif.

5. Appareil de la revendication 1 dans lequel la température de traitement est inférieure à -2,2 °C (vingt-huit degrés Fahrenheit).

6. Appareil de la revendication 1 dans lequel le réfrigérateur fait passer la température de la denrée alimentaire à pH modifié d'une température initiale supérieure au point de congélation à la température de traitement en moins de trente minutes.

7. Appareil de la revendication 1 dans lequel le dispositif de modification de pH comprend une fourniture de matière augmentant le pH et un dispositif d'application à l'intérieur duquel la denrée alimentaire initiale est exposée à la matière augmentant le pH.

8. Appareil de la revendication 7 dans lequel la matière augmentant le pH comprend du NH₃.

9. Appareil de la revendication 1 comprenant en outre :
(a) au moins un dispositif de manipulation supplémentaire, chaque dispositif de manipulation supplémentaire étant destiné à recevoir la pièce après la manipulation initiale et à manipuler la pièce de telle sorte que chaque manipulation supplémentaire par un dispositif de manipulation supplémentaire respectif produit un mouvement relatif entre les points au sein du volume de la pièce, tandis que la pièce demeure dans une gamme de températures comprenant des températures inférieures à la température de traitement.

10. Appareil de la revendication 1 dans lequel le dispositif initial de manipulation comprend :
(a) une première plaque espacée d'une seconde plaque, définissant une zone entre les plaques permettant de recevoir la pièce et une pluralité de pièces supplémentaires avec des vides entre lesdites pièces ; et
(b) un système de vérin permettant de réduire la distance entre la première plaque et la seconde plaque pour manipuler la pièce et les pièces supplémentaires contenues entre celle-ci.

11. Appareil de la revendication 9 dans lequel le dispositif initial de manipulation ou au moins un dispositif de manipulation supplémentaire comprend :
(a) un broyeur à vis pour comprimer la pièce contre une grille de broyeur, et comportant en outre une surface de coupe entraînée de façon répétitive sur la grille du broyeur.

12. Procédé permettant de réduire la teneur en microbes vivants dans une denrée alimentaire, le procédé comprenant :
(a) la modification du pH d'une denrée alimentaire initiale pour produire une denrée alimentaire à pH modifié ;
(b) le refroidissement d'une pièce à une température de traitement inférieure au point de congélation de la pièce, celle-ci comprenant un morceau de la denrée alimentaire à pH modifié ; et
(c) la manipulation de la pièce tandis que la pièce est maintenue dans une gamme de températures comprenant des températures inférieures au point de congélation de la pièce, la manipulation produisant le mouvement relatif entre les points au sein de la pièce.

13. Procédé de la revendication 12 comprenant en outre l'étape consistant à:
(a) commander la température des surfaces qui entrent en contact avec la pièce lors de la manipulation de la pièce.

14. Procédé de la revendication 12 dans lequel l'étape de modification du pH de la denrée alimentaire initiale à traiter pour produire la denrée alimentaire à pH modifié comprend :
(a) la mise en contact de la denrée initiale avec du NH₃.

15. Procédé de la revendication 12 comprenant en outre les étapes consistant à :
(a) augmenter la pression sur la pièce lors de la manipulation de celle-ci de sorte qu'au moins une partie de la pièce passe à un état décongelé lors de la manipulation de la pièce ; et
(b) replacer à l'état congelé cette partie de la pièce qui est passée à un état décongelé lors de la manipulation de la pièce.

16. Procédé de la revendication 12 dans lequel l'étape de manipulation de la pièce comprend :
(a) le passage de la pièce entre un premier rouleau et un second rouleau, le second rouleau étant espacé du premier rouleau de telle sorte que le dégagement entre le premier et le second rouleaux est inférieur à une épaisseur initiale de la pièce, si bien que la pièce est comprimée et peut s'étendre latéralement lorsqu'elle avance entre le premier et le second rouleau.

17. Procédé de la revendication 12 dans lequel l'étape de manipulation des pièces comprend :
(a) le positionnement de la pièce et d'une pluralité de pièces supplémentaires entre une première plaque et une seconde plaque ; et
(b) la diminution de la distance entre la première plaque et la seconde plaque pour comprimer la pièce et les pièces supplémentaires.

18. Procédé de la revendication 12 comprenant en outre les étapes consistant à :
(a) modifier la température de la pièce à une température différente inférieure au point de congélation de la pièce ; et
(b) manipuler la pièce dans au moins une manipulation supplémentaire tandis que la pièce est maintenue dans une gamme de températures comprenant des températures inférieures au point de congélation de la pièce, la manipulation produisant un mouvement relatif entre les points au sein de la pièce.
